# EUROPEAN PATENT SPECIFICATION

(11) **EP 1 251 797 B1**
(45) Date of publication and mention of the grant of the patent: **28.11.2007**
(21) Application number: 01905437.8
(22) Date of filing: 05.02.2001
(51) Int. Cl.: A61F 2/06

(54) **STENT INTRODUCER APPARATUS**
STENTEINFÜHRVORRICHTUNG
APPAREIL D'INTRODUCTION DE STENT

(30) Priority: 04.02.2000 US 180453 P
(43) Date of publication of application: 30.10.2002
(73) Proprietor: WILSON-COOK MEDICAL INC., Winston-Salem North Carolina 27115-4191 (US)
(72) Inventor: MOORE, Scott, T., Rural Hall, NC 27045 (US)
(74) Representative: Adams, Harvey Vaughan John
(86) International application number: PCT/US2001/003643
(87) International publication number: WO 2001/056505

(56) References cited:
- EP-A- 0 879 585
- WO-A-99/43378
- WO-A-99/44541
- US-A- 5 755 777

## Description

### Technical Field

This invention relates to medical devices, more particularly to an apparatus for delivering an implantable prosthesis.

### Background of the Invention

Placement of a stent within the biliary tree can be problematic in that the catheter delivery system must make a severe turn from the duodenum into the ostium in order to access the common bile duct. Current biliary and pancreatic stent delivery systems comprise an introducer catheter with the stent loaded at the distal end. A pusher catheter is used to deploy the stent from introducer catheter. Physicians strongly prefer that the delivery catheter be made of a clear material in order that they can see the stent within the catheter. This usually requires that the catheter be made of polytetrafluoroethylene (PTFE) which by the nature of the material, makes the catheter predisposed to kinking. When the introducer catheter kinks, it can impinge on the pusher catheter, preventing it from being able to advance the stent from the outer catheter. While the stent and pusher catheter serve to fill the lumen of the introducer catheter, making kinking within these portions less of a problem, the junction between stent and pusher is vulnerable point on the catheter where a severe kink can occur. If so, the pusher may not be able to traverse the catheter stricture to advance the stent. Some manufacturers avoid this problem because they use an axially contracting stent which overlaps with the distal end of the pusher, resulting in the most likely kinking point being reinforced by the stent and pusher from within. However, this system has other disadvantages in that stents that shorten are less desirable than non-contracting stents because of difficulty in placement. Non-shortening biliary stents, such as the ZA-STENT™ or SPIRAL Z^{™} Biliary Stents (Wilson-Cook Medical, Inc., Winston-Salem, NC), can be placed more accurately and provide superior coverage; however, the point on the catheter most susceptible to kinking is not protected by the stent, making kinking more of serious concern when PTFE is used for the introducer catheter. Another common problem with current biliary stent delivery systems is diminished recapture capability - the inability to retrieve the introducer system following stent delivery without having it become entangled within the stent or upon the introducer catheter itself. What is needed is a biliary and pancreatic stent introducer system that can still be deployed when the outer catheter kinks and that can be easily removed once the stent is deployed.

EP 0 879 585 A1 discloses a device for medical use, in particular a device for positioning a radially extendable luminal endoprosthesis in an anatomical conduit, the device comprising a tubular sheath and having a distal end and a proximal end, and within the sheath a flexible pusher. At the proximal end, the flexible pusher is held in place by a rigid stainless steel pusher. The device of document EP-0879585 comprises the features of the preamble of claim 1.

WO 99/43378 discloses a delivery system for a multi-stage stent graft.

### Summary of the Invention

The invention is as defined in the independent claim 1. Preferred embodiments are defined in the dependent claims 2-8.

The foregoing problems are solved and a technical advance is achieved in a stent introducer apparatus having a two-part pusher assembly with a lumen therethrough for introduction of a wire guide. The pusher assembly can be used to deploy a preloaded self-expanding stent from the distal end of an introducer catheter, such as a PTFE introducer sheath used to delivery a biliary or pancreatic stent. The pusher assembly comprises a first or proximal tubular portion that substantially fills the introducer catheter lumen and is made of a material with superior column strength, such as polyetheretherketone (PEEK), and a second or distal tubular portion which has a combination of good column strength and superior flexural properties, such as braided polyimide or nitinol, to distribute the severe bending force more evenly along the introducer catheter and help reduce the severity of kinking. Located at a point along the second tubular portion of the pusher assembly is a pusher member designed to urge the stent forward. The pusher member can comprise one or more separate elements attached to the second tubular portion or it can be an integral modification thereof than provides a mechanism for advancing or deploying the stent. In one configuration, the pusher member comprises a pusher head made of metal or an insert-molded polymer that provides a broad surface for applying force to advance the stent. Typically, the stent is loaded while applying pressure against the pusher head to reduce any gap therebetween and help force any kinks experienced during the procedure to occur proximal to the pusher member, thereby not interfering with the ability of the pusher assembly to advance the stent from the introducer catheter.

In a preferred embodiment of the invention, the pusher member is configured such that the proximal portion of the pusher member can more easily negotiate a kink in the introducer catheter during withdrawal of the pusher assembly following delivery. This can be accomplished by tapering the distal tubular portion. In the illustrative embodiment, a similar proximal taper occurs on the distal tip of the pusher assembly, located distal to the stent. The face of the pusher member contains a chamfer to help prevent it from digging into the inner wall of the introducer catheter. In one embodiment, there is a second member at the junction between the second tubular portion and the first tubular portion. This second member is tapered distally to help facilitate its advancement through any kink that might occur along the section of the introducer catheter that is distal to that point.

### Brief Description of the Drawing

FIG. 1 depicts a partially sectioned side view of an illustrative embodiment of the present invention;
FIG. 2 depicts a enlarged cross-sectional view of the embodiment of FIG. 1;
FIG. 3 depicts a partially sectioned view of the embodiment of FIG. 1 in a kinked introducer catheter;
FIG. 4 depicts a partially sectioned view of a second embodiment of a pusher member of the present invention;
FIG. 5 depicts a cross-sectional view of an embodiment of the present invention in which the second tubular portion extends at least substantially the length of the first tubular portion; and
FIGs. 6-7 depict cross-sectional views two embodiment of the present invention in which the first and second tubular portions or the pusher assembly comprise a single member.

### Detailed Description

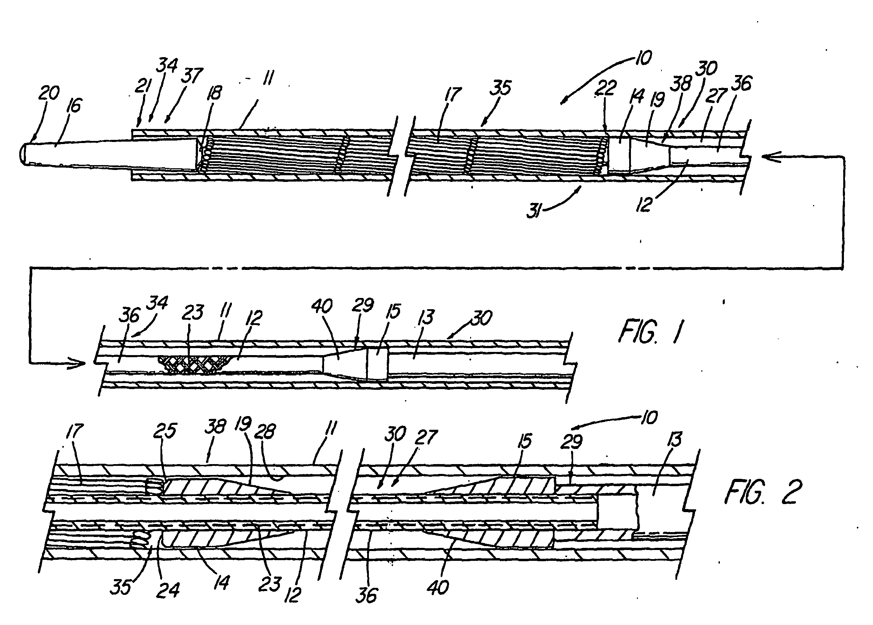

The present invention comprises a stent introducer apparatus 10, an illustrative embodiment of which is depicted in FIGs. 1-2. The stent introducer apparatus 10 comprises a pusher assembly 30 for advancing a stent 17 for deployment within a duct or vessel. In embodiment depicted in FIG. 1, the stent is a self-expanding biliary stent such as the COOK SPIRAL Z^{™} Stent; however, the type of stent is not considered important to the understanding of the invention. In the example in FIG. 1, the minimum size of the introducer catheter typically ranges from 8.0 to 8.5 FR (2.67 to 2.83 mm), depending on the stent used. The SPIRAL Z^{™} Biliary Stent, being somewhat larger than the ZA-STENT™ Biliary Stent, requires the larger introducer, while the smaller stent can be deployed from either sized introducer.

As depicted in FIGs. 1-2, the stent introducer apparatus 10 further include an introducer catheter 11, which in the illustrative embodiment, is made primarily of a substantially clear polymer such as PTFE. The pusher assembly 30 and the preloaded stent 17 are coaxially disposed within passageway 27 of the introducer catheter 11 with the stent 17 residing in the distal portion 34 of the introducer catheter until it is expelled from the distal end 21 thereof by advancement of the pusher assembly 30 or withdrawal of the introducer catheter 11.

The pusher assembly of FIGs. 1-2 comprises a first or proximal tubular portion 13 and a second or distal tubular portion 12. The first and second tubular portions 12,13 can be formed as separate members and attached, or represent different portions of a single member, each having different physical properties. Each portion 12,13 has a lumen extending therethrough that is sufficiently large for accommodating an ancillary device such as a .035" (.89 mm) wire guide. The first tubular portion 13 can comprise a rigid or non-rigid member or portion thereof, depending on the application. In the illustrative embodiment, the first tubular portion 13 comprises a non-rigid polymer tube made of a material with superior column strength. Possible materials include, but are not limited to PEEK, polyvinyl chloride (PVC), polyimide, and polyurethane. The O.D. of the first tubular portion 13, approximately .07" (1.78 mm) in the illustrative example, is such that it takes up most of the I.D. of the passageway 27 of the introducer catheter 11, thereby providing support thereto and reducing the likelihood and severity of kinking in the introducer catheter 11. Maximizing the pusher catheter O.D. also adds column strength for pushing the stent from the catheter. The second tubular portion 12 extends distally from the first tubular portion 13, to which it is joined, and comprises a tube made of a flexible material, also with sufficient column strength to allow the pusher assembly 30 to advance the stent from the introducer catheter 11. In the illustrative embodiment, the second tubular portion 12 comprises a polyimide tube reinforced with a stainless steel braid. Other possible materials include PEEK or metal tubing such as nitinol or stainless steel, depending on the degree of bending that the introducer must undergo. Nitinol tubing exhibits good laterally flexibility and kink-resistance, but is generally stiffer than braided polyimide tubing. Both the pusher assembly 30 and the introducer catheter 11 are connected at their proximal ends to a well-known coaxial medical device handle (not illustrated) that permits the pusher assembly 30 to be advanced relative to the introducer catheter 11 for deployment of the stent 17. An example of a suitable slider-type handle can be found on the previous-generation delivery systems for the Wilson-Cook SPIRAL Z^{™} and ZA-STENT™ Biliary Stents.

As a means to push the stent 17 out of the introducer catheter, a pusher member 14 is affixed to, integrally formed with the second tubular portion 12. In the illustrative embodiment, the pusher member 14 comprises a pusher head that includes a broad face 24 to contact the proximal end 31 of the stent arid urge the stent forward until deployment has been achieved. The illustrative pusher member 14 can be made of metal such as 303 or 304 stainless steel, or it can comprise a polymer that is insert molded, bonded, or otherwise attached to the second tubular portion. The O.D. of the pusher member generally depends on the type of stent to be delivered. In the illustrative example, a SPIRAL Z^{™} Biliary Stent, which is deliverable through a 8.5 Fr (2.83 mm) introducer catheter, would have a .088" (2.24 mm) O.D. pusher member 14. The ZA-STENT™ Biliary Stent, which can be introduced through either a 8.0 or 8.5 Fr ( 2.67 or 2.83 mm) introducer, could have a .077" O.D. (1.96 mm) pusher member 14 if the 8.0 Fr (2.67 mm) introducer is used. The dimensions of the pusher member 14 could vary further, depending on a number of factors, particularly the I.D. of the introducer catheter lumen 27. Because of the desirability of having the pusher member 14 diameter be as close to the I.D. of the introducer catheter lumen 27 as possible, an optional chamfer 25 is included at the outside edge of the face 24 to help prevent the pusher member 14 from digging into the inner wall 28 of the introducer catheter 11 during advancement. In the illustrative embodiment, the pusher member 14 is placed over and glued to the second tubular portion 12 such that the contact point 22 between the two lies at an intermediate point along the second tubular portion 12. In the illustrative embodiment, the pusher member 14 represents a junction 38 between two sections of the second tubular portion 12. Proximal to the pusher member 14, lies the flexible section 36 of the second tubular portion 12, while distal to the contact point 22 lies the stent loading section of the second tubular portion 12. While these two sections 35,36 comprise a single piece of reinforced polyimide tubing in the illustrative embodiment, it is also possible that they be constructed with different materials or properties insomuch that each section 35,36 is likely to experience bend stresses during introduction due to the presence of the preloaded stent 17 over the stent loading section 35. The length of the stent loading section 35 corresponds to the length of the stent 17. A distal tip 16, made of PEBAX® (Atofina Chemicals, Philadelphia, PA) or a similar soft polymer with good bonding properties, is bonded to the distal end 37 of the second tubular portion 12 after the stent 17 has been preloaded thereon. The distal tip 16 may include barium sulfate or some other agent or marker to provide radiopacity. Both the distal tip 16 and distal end 21 of the catheter are rounded for atraumatic entry into the bile duct.

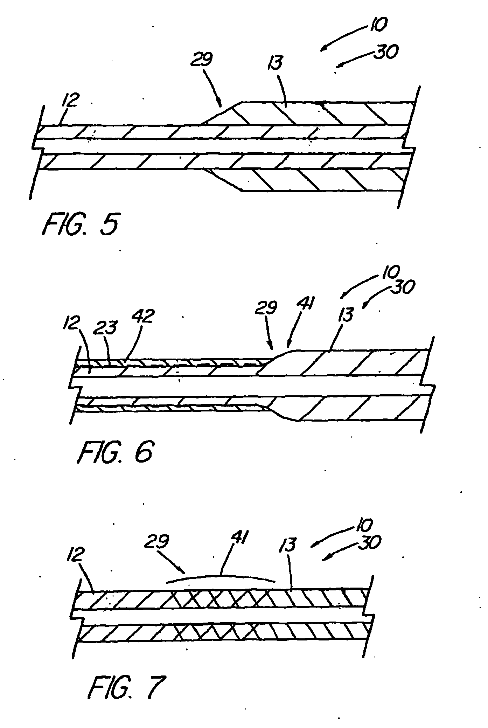

The two-part pusher assembly 30 provides an advantageous combination of both strength and flexibility that is desirable for biliary access. The section of the second tubular portion 12 proximal to the contact point 22 provides the stent introducer apparatus 10 with the ability to make a tortuous bend, such as into the ostium of the common bile duct, by distributing the bending stresses over a large area (approximately 20 cm in the illustrative embodiment). In the illustrative embodiment, the second tubular portion 12 is made to have a smaller O.D., approximately .045" (1.14 mm), to increase laterally flexibility. The first tubular portion 13 comprises the majority of the pusher assembly 30 because of the increased column strength and protection to the introducer catheter 11 it provides. For example, a pusher assembly 30 might measure 190 cm from the proximal end of the catheter (distal end of the handle) to the proximal end 31 of the stent 17, wherein 160 cm of this length might comprise the first tubular portion 13 with only 30 cm comprising the flexible section 36 of the second tubular portion 12. Generally, the flexible section should comprise about 10-20% of the pusher assembly 30 in biliary applications. For other applications, the actual length of the flexible section can be vary, depending on the application. For example, the entire stent introducer apparatus 10 could be made smaller for deploying vascular stents, or it could have utility in placing colonic stents where the anatomy can also produce severe angle that can be of concern. For biliary applications, the distance from the junction between the handle and catheter to the distal end 20 of the introducer apparatus should generally measure at least 200 cm for a typical adult patient. As shown in FIG. 2, the second tubular portion 12 is attached to the first tubular portion 13, by a well-known bonding method, such as gluing. In the illustrative embodiment, a second member 15, such as a band similar to pusher member 14, and which is made of metal or plastic, is placed at the junction 29 between the distal and first tubular portions 12,13 and glued in place with the two portions overlapping each other by approximately 3-5 mm. FIG. 5 depicts an embodiment in which the second tubular portion 12 extends the entire length (or nearly the entire length) of the first tubular portion 13 such that the latter portion is essentially providing column strength and kink resistance (especially because of the increased diameter) to the proximal or remaining portion of the pusher assembly 12 proximal to initial junction 29 point. The second tubular portion 12 can be bonded along the length of the first tubular portion 13 or affixed at one or more points, such as junction 29.

FIG. 6-7 depicts additional embodiments of the pusher assembly 30. that comprise a single continuous piece of tubing in which is modified to produce a more flexible second tubular portion 12 and a more kink-resistant first tubular portion 13. The embodiment of FIG. 6 depicts a single-piece tube in which the first tubular portion 13 is bumped down in diameter to form a thinner wall and therefore, more flexible first tubular portion 12. Extrusion techniques to vary the diameter of thermoplastic tubing are well know in the catheter arts. In the illustrative embodiment, an optional braid 23 is added to the second tubular portion 12 to allow it to be more flexible and less prone to kinking. An optional second member 15, such as that of FIG. 1, can be affixed over the transition zone 41 (or junction 29) between the two tubular portions 12,13 to facilitate negotiation of any kinks in the introducer catheter 11 that might form distal to that point. A thin layer 42 of polymer such as a shrink wrap or other type of polymer film, can be added to secure the braid 23 to the outer surface of the second tubular portion 12. In another embodiment, FIG. 7 depicts a pusher assembly 30 that has been extruded as two materials having different physical properties such as different degrees of column strength and/or flexibility. The first material, comprising the first tubular portion 13, blends with a second material comprising the second tubular portion 12 over a transition zone 41 from which the second tubular portion 12 extends distally, the second tubular portion 12 being generally more flexible than the proximal first tubular portion 13. The two materials must be compatible for co-extrusion and can include different polymers or two different compounds (e.g., different durometers) of the same polymer. Methods of co-extruding different polymers to form a single length of tubing are well known in the catheter arts.

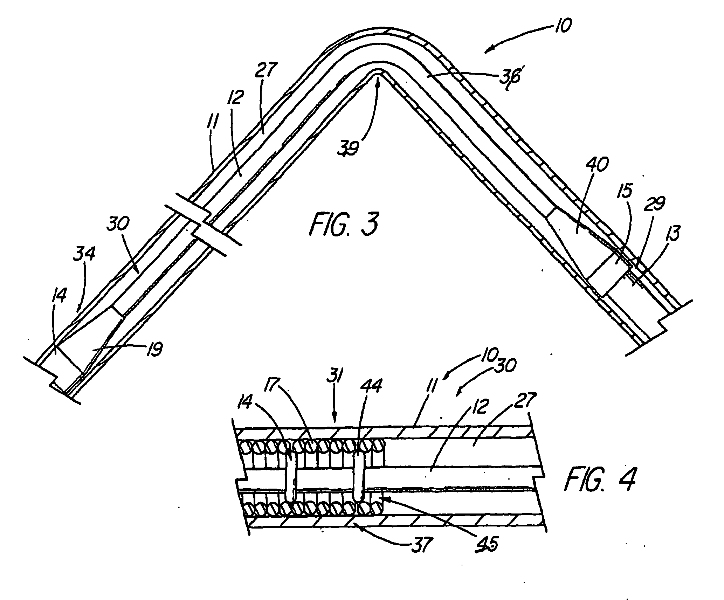

In assembling the illustrative stent introducer apparatus 10, the stent is loaded over the distal end 37 of the second tubular portion 12, and then distal tip 16 is placed thereover and bonded thereto, thereby holding the stent 17 in place. While the distal tip 16 is being affixed to the pusher assembly 30, pressure is applied such that the proximal end 31 of the stent 17 is forced tightly against the face 24 of the pusher member 14. This virtually eliminates any gap at the contact point 22, a gap which otherwise becomes a likely point of kinking when the introducer catheter is navigated through a severe bend, such as the common bile duct. The kink 39 generally occurs at that point along the introducer catheter 11 which experiences the greatest lateral bending forces during severe bending, this being largely determined by the degree of support provided by indwelling devices such as the pusher assembly 30 and the stent 17 itself. By reducing the weakness found at the contact 22 point between the pusher member 14 and the stent 17, the most likely location of any kink 39 (FIG. 3) in the introducer catheter 11 will be the flexible section 36 of the second tubular portion 12 which lies between junction 29 and the proximal end 31 of the stent 17. If a kink 39 develops within that section, it generally does not interfere with the ability of the pusher assembly 30 to slide within the introducer catheter 11 and expel the stent 17 therefrom. This is due to the pusher member 14 being distal to the kink 39 and in the case of the illustrative embodiment, the second tubular portion 12 is of a sufficiently small diameter such that the restriction of the introducer catheter lumen 27 still permits movement therethrough. Because this particular section of the introducer catheter 30 is flexible over an extended portion, any kink 39 that might occur is usually less severe than would be experienced in delivery systems of designs where the pusher system is stiff in comparison, and most of the bending force would be thus concentrated at the vulnerable contact point between the stent and the pusher member. Note that the distal surface 40 of the second member 15 is shaped to enable the second member to open the kink to permit passage therethrough of the pusher assembly.

The stent introducer apparatus 10 of FIGs. 1-2 is designed to facilitate recapture, i.e., removal of the pusher assembly 30 back through the deployed stent. A number of points on a typical introducer apparatus have the potential of snagging and catching a strut, or otherwise becoming ensnared in the stent after delivery. To reduce the possibility of this occurring in the present invention, the proximal surface includes a taper 18 that has been added to the distal tip 16 of the stent pusher assembly 30. In addition, proximal surface 19 of the pusher member 14 is also tapered as well. These tapers not only reduce the likelihood of an edge catching the stent during withdrawal, in the normal situation where the introducer catheter 11 is advanced by the physician after deployment to "recapture" the pusher assembly 30, but the tapers 18,19 also help guide the introducer catheter 11 over the distal tip 16 and pusher member 14 rather than having the distal end 21 of the introducer catheter 11 becoming temporarily caught up. In addition, the proximal tapers 18, 19 especially that of the pusher member 14, help provide a guide to traverse any strictures during withdrawal of the pusher assembly 30 if the introducer catheter 11 becomes kinked. It should be understood that the invention includes other shapes or modifications of the proximal surfaces 18,19 of the distal tip and pusher member, other than a simple taper, that would produce a surface or edge that has a reduced likelihood of catching on the stent.

While the illustrative embodiment includes an expandable stent such as the SPIRAL Z^{™} Biliary Stent, knowledge of the type of stent to be used with the present invention, or how it is delivered is not essential for an understanding of the invention. Although the illustrative embodiment depicts a pusher member 14 to urge the stent 17 from the introducer catheter 11, alternative embodiments of the present invention could include a modified pusher assembly 30 that engages with the stent in another manner rather than pushing against the proximal end 31 of the stent 17. For example, the second tubular portion could extend into the lumen of the loaded stent and be frictionally engaged therewith. For example, FIG. 4 depicts a second embodiment of pusher member 14 that urges the stent 17 forward by engaging the struts or coils of the stent 17 from inside the stent lumen 45 via one or more engagement members 44 affixed over the shaft of the second tubular member 12. These engagement members can be made of plastic or metal and vary in shape, number, and distribution along the stent loading portion 35 of the second tubular portion 12. When the stent 17 is deployed and expands, the engagement members 44 no longer engage the stent 17, permitting withdrawal of the pusher member 30. Other embodiments could include a releasable engagement mechanism between the pusher assembly 30 and stent 17.

## Claims

1. Apparatus for introducing a stent (17) into a vessel of a patient, said apparatus comprising an introducer catheter (11) having a distal portion (34);
a pusher assembly (30) having a proximal region (13) and a distal region (12), and including a pusher member (14) in the distal region for urging the stent disposed within the distal portion of the introducer catheter out of the introducer catheter, the pusher assembly being mounted within the introducer catheter and being controllable from the proximal region of the apparatus in order to exert a force on the pusher member to thereby apply an urging force against a proximal end (31) of the stent, the distal region (12) of the pusher assembly (30) having a greater ability to laterally flex in comparison to the proximal region (13) of the pusher assembly (30) **characterized in that** the said proximal region comprises the majority of the length of the pusher assembly.

2. Apparatus according to claim 1, wherein the distal region (12) of the pusher assembly (30) extends from the distal end of the proximal region (13) to the distal end of the distal region.

3. Apparatus according to claims 1 or 2, wherein any tendency of the distal portion (34) of the introducer catheter (11) to kink or remain kinked during withdrawal of the pusher assembly (30) is compensated for by shaping the proximal surface (19) of the pusher member (14).

4. Apparatus according to any one of the proceeding claims, wherein the distal region (12) includes a second member (15) at the proximal end of the distal region to engage the distal end of the proximal region (13).

5. Apparatus according to claim 4, wherein the surface of the second member (15) is shaped to enable the latter to open a kink in the catheter (11) and allow passage therethrough during movement of the pusher assembly (30) in a proximal to distal direction.

6. Apparatus according to claims 4 or 5, wherein the second member (15) is fixed to the proximal end of the distal region (12) of the pusher assembly (30), the outer part of the proximal region (13) of the pusher assembly (30) conforming to the inner diameter of the introducer catheter (11) and thereby preventing kinking at any position proximal of the second member.

7. Apparatus according to claim 6, wherein the distal region (12) of the pusher assembly (30) further has an outer diameter significantly smaller than the outer diameter of the proximal region (13) of the pusher assembly.

8. Apparatus according to any one of the preceding claims, wherein the distal face (24) of the pusher member (14) is chamfered to facilitate its movement within the introducer catheter (11).

## Patentansprüche

1. Gerät zur Einführung eines Stents (17) in ein Gefäß eines Patienten, wobei das Gerät einen Einführkatheter (11) mit einem distalen Abschnitt (34) und eine Schubanordnung (30) mit einer proximalen Region (13) und einer distalen Region (12) umfasst, **dadurch gekennzeichnet, dass** die proximale Region den größten Teil der Länge der Schubanordnung umfasst und ein Schubelement (14) in der distalen Region aufweist, um den im distalen Abschnitts des Einführkatheters angeordneten Stents aus dem Einführkatheter zu schieben, wobei die Schubanordnung im Einführkatheter befestigt ist und von der proximalen Region des Geräts aus gesteuert werden kann, um eine Kraft auf das Schubelement auszuüben und damit eine Schubkraft gegen ein proximales Ende (31) des Stents aufzubringen, wobei die distale Region (12) der Schubanordnung (30) sich seitlich stärker biegen kann als die proximale Region (13) der Schubanordnung (30).

2. Gerät nach Anspruch 1, worin die distale Region (12) der Schubanordnung (30) sich vom distalen Ende der proximalen Region (13) zum distalen Ende der distalen Region erstreckt.

3. Gerät nach Anspruch 1 oder 2, worin eine Tendenz des distalen Abschnitts (34) des Einführkatheters (11), beim Herausziehen der Schubanordnung (30) zu knicken oder geknickt zu bleiben, durch Formung der proximalen Fläche (19) des Schubelements (14) ausgeglichen wird.

4. Gerät nach einem der vorhergehenden Ansprüche, worin die distale Region (12) ein zweites Element (15) am proximalen Ende der distalen Region aufweist, um in das distale Ende der proximalen Region (13) einzugreifen.

5. Gerät nach Anspruch 4, worin die Oberfläche des zweiten Elements (15) so geformt ist, dass sie es dem zweiten Element (15) besser ermöglicht, einen Knick im Katheter (11) zu öffnen und den Durchgang dort hindurch bei der Bewegung der Schubanordnung (30) in einer Richtung von proximal nach distal zu gestatten.

6. Gerät nach Anspruch 4 oder 5, worin das zweite Element (15) am proximalen Ende der distalen Region (12) der Schubanordnung (30) befestigt ist, wobei der äußere Teil der proximalen Region (13) der Schubanordnung (30) sich an den Innendurchmesser des Einführkatheters (11) anpasst und so Knicken an jeder Stelle proximal vom zweiten Element verhindert.

7. Gerät nach Anspruch 6, worin die distale Region (12) der Schubanordnung (30) ferner einen Außendurchmesser aufweist, der signifikant kleiner ist als der Außendurchmesser der proximalen Region (13) der Schubanordnung.

8. Gerät nach einem der vorhergehenden Ansprüche, worin die distale Fläche (24) des Schubelements (14) abgeschrägt ist, um seine Bewegung im Einführkatheter (11) zu erleichtern.

## Revendications

1. Appareil pour introduire un stent (17) dans un vaisseau d'un patient, ledit appareil comprenant un cathéter introducteur (11) ayant une partie distale (34);
un ensemble poussoir (30) ayant une région proximale (13) et une région distale (12) et comprenant un membre poussoir (14) dans la région distale pour forcer le stent, disposé dans la partie distale du cathéter introducteur, hors du cathéter introducteur, l'ensemble poussoir étant monté dans le cathéter introducteur et étant contrôlable de la région proximale de l'appareil afin d'exercer une force sur le membre poussoir pour appliquer ainsi une force de poussée contre une extrémité proximale (31) du stent, la région distale (12) de l'ensemble poussoir (30) ayant une plus grande aptitude à la flexion latérale comparée à la région proximale (13) de l'ensemble poussoir (30), **caractérisé en ce que** ladite région proximale comprend la majorité de la longueur de l'ensemble poussoir.

2. Appareil selon la revendication 1, dans lequel la région distale (12) de l'ensemble poussoir (30) s'étend de l'extrémité distale de la région proximale (13) jusqu'à l'extrémité distale de la région distale.

3. Appareil selon les revendications 1 ou 2, dans lequel toute tendance de la partie distale (34) du cathéter introducteur (11) à faire une boucle ou à rester bouclée pendant le retrait de l'ensemble poussoir (30) est compensée en façonnant la surface proximale (19) du membre poussoir (14).

4. Appareil selon l'une quelconque des revendications précédentes, dans lequel la région distale (12) comprend un deuxième membre (15) à l'extrémité proximale de la région distale pour s'engager avec l'extrémité distale de la région proximale (13).

5. Appareil selon la revendication 4, dans lequel la surface du deuxième membre (15) est façonnée pour permettre à ce dernier d'ouvrir une boucle dans le cathéter (11) et permettre le passage à travers celui-ci pendant le mouvement de l'ensemble poussoir (30) d'une direction proximale à une direction distale.

6. Appareil selon les revendications 4 ou 5, dans lequel le deuxième membre (15) est fixé à l'extrémité proximale de la région distale (12) de l'ensemble poussoir (30), la partie externe de la région proximale (13) de l'ensemble poussoir (30) se conformant au diamètre interne du cathéter introducteur (11) et empêchant ainsi la formation d'une boucle à une position proximale quelconque du deuxième membre.

7. Appareil selon la revendication 6, dans lequel la région distale (12) de l'ensemble poussoir (30) a en outre un diamètre externe significativement plus petit que le diamètre externe de la région proximale (13) de l'ensemble poussoir.

8. Appareil selon l'une quelconque des revendications précédentes, dans lequel la face distale (24) du membre poussoir (14) est chanfreinée pour en faciliter le mouvement dans le cathéter introducteur (11).
